# EUROPÄISCHE PATENTSCHRIFT

(11) **EP 2 729 252 B1**
(45) Veröffentlichungstag und Bekanntmachung des Hinweises auf die Patenterteilung: **09.08.2017**
(21) Anmeldenummer: 12734846.4
(22) Anmeldetag: 22.06.2012
(51) Int. Cl.: B01L 3/00, B01L 9/06, G01F 23/24

(54) **KÜVETTENMODUL MIT ELEKTRISCH LEITFÄHIGEM KÜVETTENTRÄGER**
CUVETTE MODULE WITH ELECTRICALLY CONDUCTIVE CUVETTE HOLDER
MODULE DE CUVETTE DOTÉ D'UN SUPPORT DE CUVETTE CONDUCTEUR ÉLECTRIQUE

(30) Priorität: 07.07.2011 EP 11173047
(43) Veröffentlichungstag der Anmeldung: 14.05.2014
(73) Patentinhaber: Behnk, Holger, 22417 Hamburg (DE)
(72) Erfinder: BEHNK, Holger, 22417 Hamburg (DE)
(74) Vertreter: Glawe, Delfs, Moll
(86) Internationale Anmeldenummer: PCT/EP2012/062159
(87) Internationale Veröffentlichungsnummer: WO 2013/004523

(56) Entgegenhaltungen:
- EP-A1- 1 867 986
- EP-A2- 1 116 953
- EP-A2- 1 992 952
- DE-A1-102008 043 412
- US-A- 5 315 872
- US-A1- 2002 172 789
- US-A1- 2009 004 064

## Beschreibung

Die Erfindung betrifft ein Küvettenmodul mit einem Küvettenträger und einer von dem Küvettenträger getragenen Küvette. In dem Küvettenträger ist eine Zwischenkammer ausgebildet. Die Erfindung betrifft außerdem ein Verfahren zum Übertragen einer vorgegebenen Flüssigkeitsmenge aus einem Transportbehälter in eine Küvette.

Solche Küvettenmodule können bei der Analyse von Flüssigkeiten, insbesondere bei der Analyse von Körperflüssigkeiten verwendet werden. Für die Analyse ist es erforderlich, eine genau abgemessene Menge der Flüssigkeit aus einem Transportbehälter in die Küvette zu übertragen. Da die Transportbehälter unter Überdruck oder Unterdruck stehen können, ist es nicht ganz einfach, eine definierte Flüssigkeitsmenge direkt aus dem Transportbehälter heraus zu entnehmen. Dies gilt insbesondere, wenn es sich um kleine Flüssigkeitsmengen handelt. Es ist deswegen bekannt, die Flüssigkeit zunächst in eine Zwischenkammer einzubringen und die Flüssigkeitsmenge aus der Zwischenkammer heraus präzise abzumessen, siehe EP 1 867 986.

Dass die vorgegebene Flüssigkeitsmenge aus der Zwischenkammer heraus entnommen werden kann, setzt voraus, dass die Flüssigkeitsmenge, die zuvor aus dem Transportbehälter in die Zwischenkammer übertragen wurde, größer ist als die vorgegebene Flüssigkeitsmenge. Vor der Entnahme der vorgegebenen Flüssigkeitsmenge aus der Zwischenkammer wird deswegen der Füllstand in der Zwischenkammer gemessen. Nur wenn genügend Flüssigkeit in der Zwischenkammer enthalten ist, kann die vorgegebene Flüssigkeitsmenge entnommen werden. Ist zu wenig Flüssigkeit in der Zwischenkammer, ist dies ein Hinweis auf einen Fehler.

Eine Möglichkeit, den Füllstand in dem Zwischenbehälter zu messen, besteht darin, einen elektrischen Sensor an die Oberfläche der Flüssigkeit heranzuführen und aus einer Änderung des elektrischen Signals darauf zu schließen, dass der Sensor in die Flüssigkeit eingetaucht ist. Wenn die Zwischenkammer, was regelmäßig der Fall ist, aus einem Kunststoffmaterial besteht, kommt es dabei immer wieder zu verfälschten Messergebnissen.

Der Erfindung liegt die Aufgabe zu Grunde, ein Küvettenmodul und ein zugehöriges Verfahren vorzustellen, mit denen das Abmessen kleiner Flüssigkeitsmengen erleichtert wird. Ausgehend vom eingangs genannten Stand der Technik wird die Aufgabe gelöst mit den Merkmalen der unabhängigen Ansprüche. Erfindungsgemäß besteht der Küvettenträger aus einem elektrisch leitfähigen Kunststoffmaterial. Die Küvette besteht aus einem anderen Material als der Küvettenträger, wobei die Benetzbarkeit des Küvettenträgers höher ist als die Benetzbarkeit der Küvette. Vorteilhafte Ausführungsformen finden sich in den Unteransprüchen.

Zunächst werden einige Begriffe erläutert. Eine Küvette bezeichnet im Rahmen der Erfindung einen Behälter, in den eine abgemessene Menge Flüssigkeit eingebracht werden kann, um an der Flüssigkeit Analysen durchzuführen. Bei der Flüssigkeit kann es sich um eine Körperflüssigkeit handeln. Eine mögliche Anwendung ist das Messen der Blutgerinnungszeit, siehe EP 0 369 168.

Der Begriff Zwischenkammer bezeichnet eine Struktur des Küvettenträgers, die bei geeigneter Ausrichtung der Kammer eine Flüssigkeit aufnehmen kann. Die Zwischenkammer ist regelmäßig nicht zu allen Seiten hin abgeschlossen, die Flüssigkeit fließt also aus der Zwischenkammer heraus, wenn das Küvettenmodul auf den Kopf gestellt wird. Die Zwischenkammer ist ein Bestandteil des Küvettenträgers, was bedeutet, dass die Wände der Zwischenkammer aus elektrisch leitfähigem Kunststoff bestehen. Durch den Bestandteil "Zwischen" wird zum Ausdruck gebracht, dass die Zwischenkammer dazu verwendet werden kann, eine Flüssigkeit zwischenzeitlich aufzunehmen. Eine strukturelle Einschränkung ist damit nicht verbunden.

Der erfindungsgemäße Vorteil besteht darin, dass durch die elektrische Leitfähigkeit des Kunststoffmaterials Störsignale vermieden werden. Es wird deswegen möglich, durch Eintauchen eines elektrischen Sensors zu ermitteln, welchen Füllstand die Flüssigkeit in der Zwischenkammer hat. Es kann damit auf einfache Art sichergestellt werden, dass ausreichend Flüssigkeit in der Zwischenkammer enthalten ist, bevor die vorgegebene Flüssigkeitsmenge entnommen wird und in die Küvette übertragen wird.

Kunststoffe sind im Allgemeinen Isolatoren, die keine oder eine nur sehr geringe elektrische Leitfähigkeit haben. Um ausgehend von einem solchen Kunststoff zu einem elektrisch leitfähigen Kunststoffmaterial zu gelangen, wie es für die Erfindung geeignet ist, kann das Kunststoffmaterial mit elektrisch leitfähigen Partikeln gefüllt werden. Um eine hohe Leitfähigkeit zu erreichen, werden die Partikel dem Kunststoffmaterial vorzugsweise in einer Konzentration zugefügt, dass die Partikel nur einen geringen Abstand zeinander haben oder einander sogar berühren. Das elektrisch leitfähige Kunststoffmaterial ist vorzugsweise so eingestellt, dass der spezifische Widerstand des Materials bei Raumtemperatur kleiner ist als 10⁶ Ω cm.

Wenn elektrische Störsignale vermieden werden sollen, sollte insbesondere der Oberflächenwiderstand klein sein. Der Oberflächenwiderstand bezeichnet den Widerstand, den man zwischen zwei auf die Oberfläche des Materials aufgelegten Elektroden misst. Der Oberflächenwiderstand hängt von der Geometrie des Küvettenträgers und der Positionierung der Elektroden auf dem Küvettenträger ab. Im Rahmen der Erfindung beziehen sich die Werte für den Oberflächenwiderstand auf eine Messung nach der folgenden Vorschrift. In zwei nebeneinander liegende Zwischenkammern wird jeweils eine metallene Elektrode eingesteckt, die mit drei Wänden der Zwischenkammer in flächigem Kontakt steht. Die drei Wände können zwei Seitenwände und der Boden sein. Der elektrische Widerstand zwischen den beiden Elektroden wird gemessen. Der Oberflächenwiderstand gemäß dieser Messung liegt vorzugsweise zwischen 1 kΩ und 50 kΩ, weiter vorzugsweise zwischen 5 kΩ und 30 kΩ, weiter vorzugsweise zwischen 9 kΩ und 14 kΩ.

Von der Füllhöhe in der Zwischenkammer kann nur dann zuverlässig auf die Flüssigkeitsmenge geschlossen werden, wenn die Zwischenkammer vollständig durch die Flüssigkeit benetzt ist. Im Allgemeinen ist die Benetzbarkeit der Oberfläche bei Kunststoffmaterialien, wie sie als Ausgangsmaterial für den elektrisch leitfähigen Kunststoff verwendet werden, nicht besonders ausgeprägt. Wasser bildet also ausgeprägte Tropfen auf der Oberfläche, anstatt sich flächig zu verteilen. Bei schlechter Benetzbarkeit der Oberfläche des elektrisch leitfähigen Kunststoffs besteht das Risiko, dass sich Lufteinschlüsse in der Zwischenkammer bilden, so dass aus der Füllhöhe nicht mehr auf die Flüssigkeitsmenge geschlossen werden kann. Es hat sich gezeigt, dass die Benetzbarkeit des Kunststoffmaterials verbessert werden kann, wenn ein geeignetes leitfähiges Füllmaterial verwendet wird. Insbesondere kommen als Füllmaterial Graphit oder leitfähige Rußpartikel, so genannter Leitruß, in Betracht. Wenn diese Partikel bis an die Oberfläche des Kunststoffmaterials reichen, also ein Teil der Oberfläche von diesen Partikeln gebildet wird, hat dies eine erheblich verbesserte Benetzbarkeit zur Folge.

Bei der Küvette, in die die Flüssigkeit aus der Zwischenkammer heraus übertragen wird, ist hingegen eine hohe Benetzbarkeit gerade nicht gewünscht. Während der Analyse wird die Flüssigkeit nämlich meist mit einer zweiten Flüssigkeit vermischt. Um zu vermeiden, dass sich die Flüssigkeiten bereits vor der Analyse vermischen, ist es von Vorteil, wenn die Flüssigkeit möglichst ausgeprägte Tropfen auf der Oberfläche bildet. Die Küvette besteht deswegen aus einem anderen Material als der Küvettenträger, wobei die Benetzbarkeit des Küvettenträgers höher ist als die Benetzbarkeit der Küvette. Die Benetzbarkeit kann gemessen werden, indem der Winkel, den ein Tropfen mit der Oberfläche einschließt, gemessen wird. Der Benetzungswinkel der Küvette ist vorzugsweise um mindestens 10°, weiter vorzugsweise um mindestens 30°, weiter vorzugsweise um mindestens 45° größer als der Benetzungswinkel des Küvettenträgers.

Das erfindungsgemäße Küvettenmodul wird regelmäßig bei der Analyse kleiner Flüssigkeitsmengen verwendet. Die Dimension des Küvettenmoduls sollte den Flüssigkeitsmengen angepasst sein. So hat die Zwischenkammer vorzugsweise ein Volumen zwischen 10 µl und 500 µl, weiter vorzugsweise ein Volumen zwischen 50 µl und 250 µl. Das Küvettenmodul kann so ausgelegt sein, dass in einem Durchgang mehrere Analysen durchgeführt werden können. Das Küvettenmodul kann dazu eine Mehrzahl von Zwischenkammern und eine Mehrzahl von Küvetten umfassen. Vorzugsweise ist die Anzahl der Zwischenkammern gleich der Anzahl der Küvetten. Um die automatische Handhabung des Küvettenmoduls zu erleichtern, kann das Küvettenmodul mit einer Zahnung versehen sein, in die ein Zahnrad einer Maschine eingreifen kann.

Die Erfindung betrifft außerdem ein Verfahren zum Übertragen einer vorgegebenen Flüssigkeitsmenge aus einem Transportbehälter in eine Küvette. Bei dem Verfahren wird zunächst eine Flüssigkeitsmenge, die größer ist als die vorgegebene Flüssigkeitsmenge, aus dem Transportbehälter in eine Zwischenkammer übertragen, wobei die Zwischenkammer aus einem elektrisch leitfähigen Kunststoffmaterial besteht. Es wird der Füllstand in der Zwischenkammer ermittelt, indem ein elektrischer Sensor in die Zwischenkammer eingeführt wird und eine Veränderung des elektrischen Signals festgestellt wird, wenn der Sensor in die Flüssigkeit eintaucht. Es wird überprüft, ob die Flüssigkeitsmenge in der Zwischenkammer größer ist als die vorgegebene Flüssigkeitsmenge und eine Fehlermeldung gegeben, wenn dies nicht der Fall ist. Die vorgegebene Flüssigkeitsmenge wird anschließend aus der Zwischenkammer entnommen und in die Küvette übertragen.

Die Flüssigkeitsmenge kann mittels einer Hohlnadel aus der Zwischenkammer entnommen werden. Die Hohlnadel kann gleichzeitig als elektrischer Sensor dienen. Die Hohlnadel kann Bestandteil eines bei Resonanzfrequenz betriebenen Schwingkreises sein. Das Schwingverhalten ändert sich, wenn die Hohlnadel in die Flüssigkeit eingetaucht wird. Das Verfahren kann mit weiteren Merkmalen fortgebildet werden, die oben mit Bezug auf das erfindungsgemäße Küvettenmodul beschrieben sind.

Die Erfindung wird nachfolgend unter Bezugnahme auf die beigefügten Zeichnungen anhand vorteilhafter Ausführungsformen beispielhaft beschrieben. Es zeigen:
- Fig. 1:: eine perspektivische Ansicht eines erfindungsgemäßen Küvettenmoduls;
- Fig. 2:: das Küvettenmodul aus Fig. 1 in einer Ansicht von oben;
- Fig. 3:: eine schematische Querschnittsansicht einer Küvette in zwei verschiedenen Stellungen;
- Fig. 4:: zwei unterschiedliche Benetzungswinkel;
- Fig. 5:: eine schematische Darstellung einer Füllstandsmessung in der Zwischenkammer; und
- Fig. 6:: eine schematische Darstellung eines Verfahrens zum Messen des Oberflächenwiderstands.

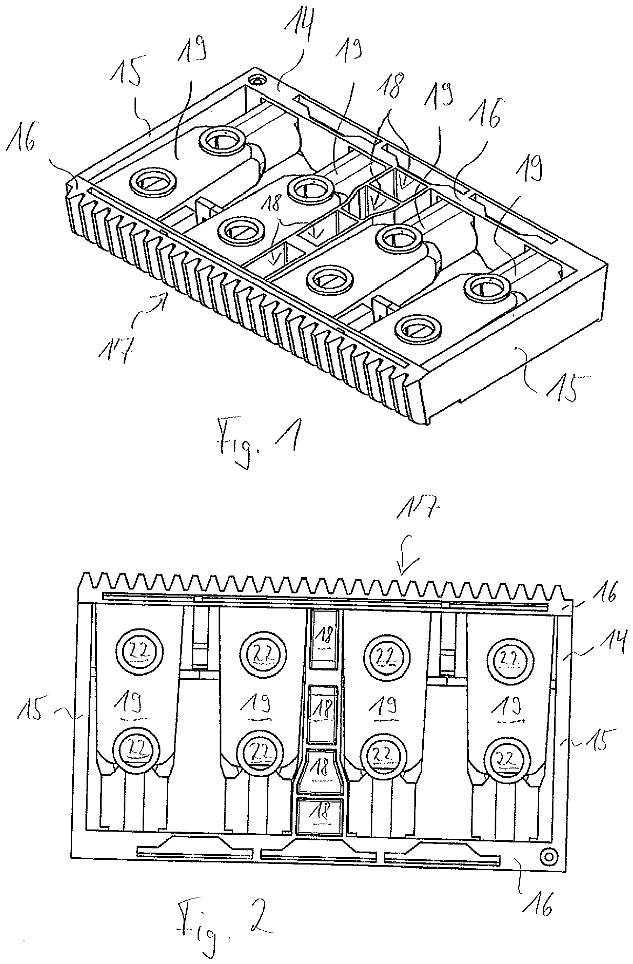

Ein erfindungsgemäßes Küvettenmodul in Fig. 1 umfasst einen Küvettenträger 14, der einen aus zwei Querstegen 15 und zwei Längsstegen 16 zusammengesetzten äußeren Rahmen bildet. An einem der Längsstege 16 ist eine Zahnung 17 ausgebildet, über die das Küvettenmodul präzise positioniert werden kann, wenn es in einer Schiene geführt ist.

In der Mitte zwischen den beiden Querstegen 15 umfasst der Küvettenträger 14 eine Anordnung von vier Zwischenkammern 18. Der Küvettenträger 14 mit den beiden Querstegen 15, den beiden Längsstegen 16 und den Zwischenkammern 18 ist einstückig ausgebildet und besteht aus einem elektrisch leitfähigen Kunststoffmaterial. Die Leitfähigkeit wird dadurch erreicht, dass ein nicht leitfähiges Kunststoffausgangsmaterial mit einem hinreichenden Anteil von leitfähigen Russpartikeln gefüllt wird.

Der Küvettenträger 14 ist außerdem mit Aufnahmen für vier Küvetten 19 ausgestattet. Die vier Küvetten 19 sind als Spritzgussteil hergestellt und bestehen aus einem für solche Zwecke gebräuchlichen Kunststoffmaterial. Die vier Küvetten 19 sind über einen in Fig. 1 nicht sichtbaren Verbindungssteg miteinander verbunden und werden als Einheit in die Aufnahme des Küvettenträgers 14 eingerastet. Sowohl die Küvette 19 als auch der Küvettenträger 14 bestehen vorzugsweise aus einem Material, das mit Körperflüssigkeiten wie Blut nicht reagiert.

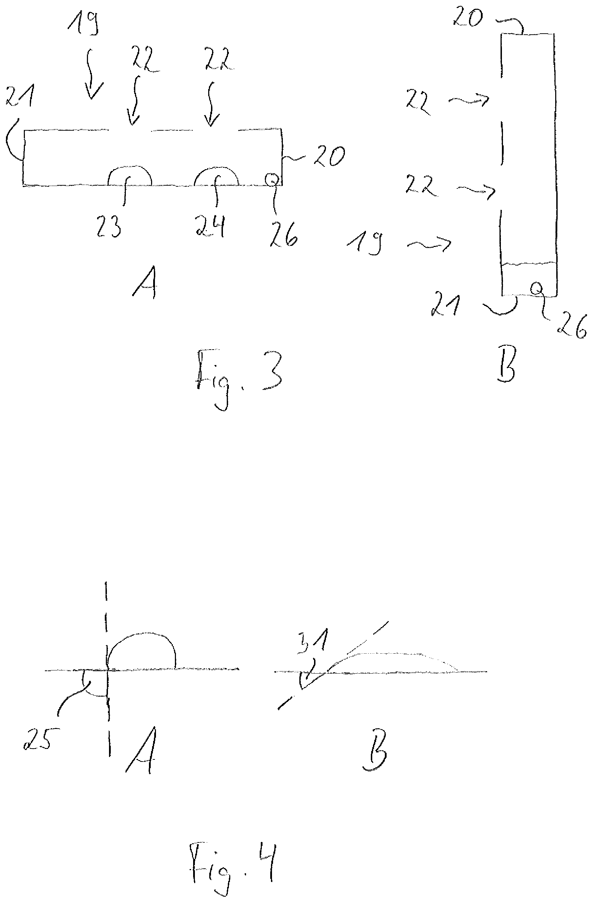

Die Küvetten 19 erstrecken sich gemäß Fig. 3 jeweils von einem oberen Ende 20, das in Fig. 1 in Richtung der Zahnung 17 weist, bis zu einem unteren Ende 21, das an den gegenüberliegenden Längssteg 16 angrenzt. Jede Küvette 19 weist zwei Öffnungen 22 auf, durch die kleine Flüssigkeitsmengen 23, 24 in die Küvette 19 eingebracht werden können, wenn die Küvette wie in Fig. 3A in liegender Position angeordnet ist. Das Kunststoffmaterial der Küvette 19 hat eine geringe Benetzbarkeit, so dass die Flüssigkeitsmengen 23, 24 ausgeprägte Tropfen auf der Oberfläche des Kunststoffmaterials bilden. Die Tropfen haben einen großen Benetzungswinkel 25, der gemäß Fig. 4A etwa 90° beträgt.

Wird die Küvette 19 um 90° in die in Fig. 3B gezeigte Position geschwenkt, fließen die Flüssigkeitsmengen 23, 24 an der Wand entlang nach unten und sammeln sich am unteren Ende 21 der Küvette 19. Eine in der Küvette 19 enthaltene Kugel 26 rollt ebenfalls nach unten und kann dazu dienen, die Flüssigkeitsmengen 23, 24 für die Analyse durch ein nicht dargestelltes Magnetrührwerk gleichmäßig zu vermischen. Die Flüssigkeitsmenge 23 kann beispielsweise Blut und die Flüssigkeitsmenge 24 ein Reagenz sein und die Analyse kann darin bestehen, die Gerinnungszeit zu messen.

Das Blut wird normalerweise in einem nicht dargestellten geschlossenen Transportbehälter bereitgestellt, in dem eine größere Flüssigkeitsmenge enthalten ist, als für die Analyse benötigt wird. Da in dem Transportbehälter ein Überdruck oder Unterdruck anliegen kann, ist es nicht ohne weiteres möglich, die für die Analyse benötigte kleine Flüssigkeitsmenge 23 direkt aus dem Transportbehälter heraus präzise abzumessen. Es wird deswegen zunächst eine Flüssigkeitsmenge, die größer ist als die vorgegebene Flüssigkeitsmenge 23 aus dem Transportbehälter entnommen und in eine der Zwischenkammern 18 des Küvettenträgers 14 eingebracht.

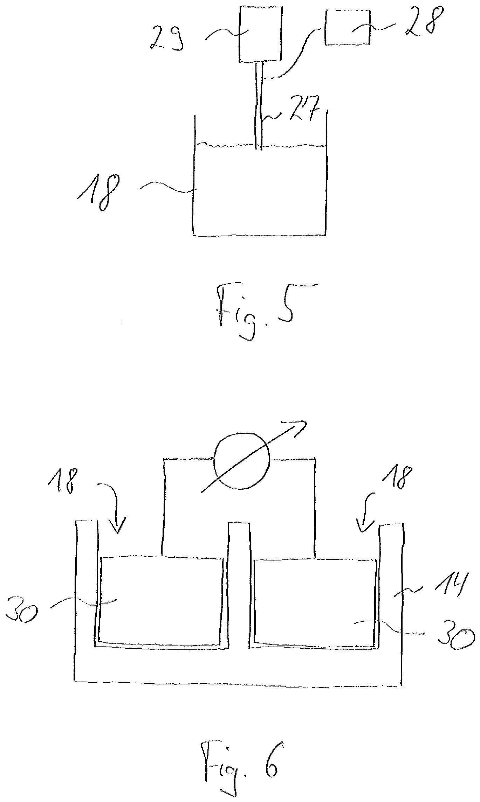

Vor der Entnahme der vorgegebenen Flüssigkeitsmenge 23 aus der Zwischenkammer 18 wird überprüft, ob die in der Zwischenkammer 18 enthaltene Flüssigkeitsmenge tatsächlich größer ist als die vorgegebene Flüssigkeitsmenge 23. Es wird dazu gemäß Fig. 5 eine Hohlnadel 27 von oben in die Zwischenkammer 18 eingeführt. Die Hohlnadel 27 ist an einen in Fig. 5 nur schematisch dargestellten elektrischen Schaltkreis 28 angeschlossen, der eine Veränderung des elektrischen Signals registriert, wenn die Hohlnadel 27 in die Flüssigkeit eingetaucht. Aus dem Füllstand ergibt sich, ob in der Zwischenkammer 18 eine ausreichende Flüssigkeitsmenge enthalten ist. Ist dies nicht der Fall, wird eine Fehlermeldung gegeben. Ist genügend Flüssigkeit in der Zwischenkammer 18 enthalten, wird mittels einer Abmesseinrichtung 29, an die die Hohlnadel 27 angeschlossen ist, die vorgegebene Flüssigkeitsmenge 23 aus der Zwischenkammer 18 entnommen. Die Hohlnadel 27 wird dann eine der Öffnungen 22 der Küvette 19 eingeführt und die vorgegebene Flüssigkeitsmenge 23 wird wieder abgegeben.

Die Zwischenkammer 18 hat eine Kapazität von 150 µl, die abzumessende Flüssigkeitsmenge 23 kann beispielsweise 20 µl betragen. Bei derart kleinen Flüssigkeitsmengen besteht ein Risiko, dass das elektrische Signal verfälscht wird, anhand dessen das Eintauchen der Hohlnadel 27 in die Flüssigkeit festgestellt wird. Gemäß der Erfindung ist deswegen vorgesehen, dass der Küvettenträger 14 und damit die Wände der Zwischenkammer 18 aus einem elektrisch leitfähigen Kunststoff bestehen. Es zeigt sich, dass es dadurch möglich wird, die Veränderung des elektrischen Signals beim Eintauchen der Hohlnadel 27 zuverlässig festzustellen.

Als Kriterium, ob ein elektrisch leitfähiges Kunststoffmaterial als Material für den Küvettenträger 14 geeignet ist, kann eine Messung des Oberflächenwiderstands durchgeführt werden. Es wird dazu in zwei nebeneinanderliegende Zwischenkammern 18 jeweils eine metallene Elektrode 30 eingeführt, die so bemessen ist, dass sie an zwei gegenüberliegenden Seitenwänden und dem Boden der Zwischenkammer 18 anliegt. Bei dieser Messung sollte der zwischen den beiden Elektroden 30 gemessene Widerstand zwischen 9 kΩ und 14 kΩ liegen.

Damit aus der in Fig. 5 gezeigten Messung des Füllstands zuverlässig auf die in der Zwischenkammer 18 enthaltene Flüssigkeitsmenge geschlossen werden kann, muss die Zwischenkammer 18 gleichmäßig mit der Flüssigkeit gefüllt sein und es dürfen keine Luftblasen eingeschlossen sein. Dazu ist es von Vorteil, wenn das elektrisch leitfähige Kunststoffmaterial eine hohe Benetzbarkeit hat. Eine auf die Oberfläche des Materials aufgetragene Flüssigkeitsmenge verteilt sich dann großflächig und bildet keinen ausgeprägten Tropfen. Der in Fig. 4B gezeigte Benetzungswinkel 31 kann beispielsweise 30° betragen und damit um 60° kleiner sein als der Benetzungswinkel 25, den die gleiche Flüssigkeitsmenge auf dem Material der Küvette 19 hat. Die hohe Benetzbarkeit des elektrisch leitfähigen Kunststoffmaterials ergibt sich daraus, dass die leitfähigen Rußpartikel so in dem Kunststoffmaterial verteilt sind, dass sie einen Teil der Oberfläche des Materials bilden.

## Patentansprüche

1. Küvettenmodul mit einem Küvettenträger (14) und einer von dem Küvettenträger (14) gehaltenen Küvette (19), wobei in dem Küvettenträger (14) eine Zwischenkammer (18) ausgebildet ist, **dadurch gekennzeichnet, dass** der Küvettenträger (14) aus einem elektrisch leitfähigen Kunststoffmaterial besteht, dass die Küvette (19) aus einem anderen Material besteht als der Küvettenträger (14) und dass die Benetzbarkeit des Küvettenträgers (14) höher ist als die Benetzbarkeit der Küvette (19).

2. Küvettenmodul nach Anspruch 1, **dadurch gekennzeichnet, dass** der spezifische Widerstand des elektrisch leitfähigen Kunststoffmaterials bei Raumtemperatur kleiner ist als 10⁶ Ω cm.

3. Küvettenmodul nach Anspruch 1 oder 2, **dadurch gekennzeichnet, dass** der Küvettenträger zwei nebeneinander liegende Zwischenkammern umfasst und dass der mit zwei metallenen Elektroden (30), die in die Zwischenkammern (18) eingesteckt sind und die jeweils mit drei Wänden der jeweiligen Zwischenkammer (18) in flächigem Kontakt stehen, gemessene Oberflächenwiderstand des Küvettenträgers (14) zwischen 1 kΩ und 50 kΩ, weiter vorzugsweise zwischen 5 kΩ und 30 kΩ, weiter vorzugsweise zwischen 9 kΩ und 14 kΩ liegt.

4. Küvettenmodul nach einem der Ansprüche 1 bis 3, **dadurch gekennzeichnet, dass** der elektrisch leitfähige Kunststoff durch Verwendung eines leitfähigen Füllmaterials aus einem Kunststoffausgangsmaterial hergestellt ist, wobei der elektrische leitfähige Kunststoff eine erhöhte Benetzbarkeit verglichen mit dem Kunststoffausgangsmaterial hat.

5. Küvettenmodul nach Anspruch 4, **dadurch gekennzeichnet, dass** der Füllstoff des elektrisch leitfähigen Kunststoffs Graphit oder Leitruß ist.

6. Küvettenmodul nach einem der Ansprüche 1 bis 5, **dadurch gekennzeichnet, dass** der Benetzungswinkel des Küvettenträgers (14) um mindestens 10°, vorzugsweise um mindestens 30°, weiter vorzugsweise um mindestens 45° kleiner ist als der Benetzungswinkel der Küvette (19) .

7. Küvettenmodul nach einem der Ansprüche 1 bis 6, **dadurch gekennzeichnet, dass** es eine Mehrzahl von Zwischenkammern (18) und eine Mehrzahl von Küvetten (19) umfasst.

8. Küvettenmodul nach einem der Ansprüche 1 bis 7, **dadurch gekennzeichnet, dass** das Volumen der Zwischenkammer (18) zwischen 1 µl und 500 µl, vorzugsweise zwischen 50 µl und 250 µl liegt.

9. Verfahren zum Übertragen einer vorgegebenen Flüssigkeitsmenge (23) aus einem Transportbehälter in eine Küvette (19) mit folgenden Schritten:
a. Übertragen einer Flüssigkeitsmenge, die größer ist als die vorgegebene Flüssigkeitsmenge (23), aus dem Transportbehälter in eine Zwischenkammer (18), wobei die Zwischenkammer (18) aus einem elektrisch leitfähigen Kunststoffmaterial besteht und wobei die Zwischenkammer (18) in einem Küvettenträger (14) ausgebildet ist;
b. Ermitteln des Füllstands in der Zwischenkammer (18), indem ein elektrischer Sensor in die Zwischenkammer (18) eingeführt wird und eine Veränderung des elektrischen Signals festgestellt wird, wenn der elektrische Sensor in die Flüssigkeit eintaucht;
c. Feststellen, ob die Flüssigkeitsmenge in der Zwischenkammer (18) größer ist als die vorgegebene Flüssigkeitsmenge (23) und Ausgeben einer Fehlermeldung, wenn dies nicht der Fall ist;
d. Entnehmen der vorgegebenen Flüssigkeitsmenge (23) aus der Zwischenkammer (18) und Übertragen der vorgegebenen Flüssigkeitsmenge (23) in die Küvette (19), wobei die Küvette (19) aus einem anderen Material besteht als der Küvettenträger (14) und wobei die Benetzbarkeit des Küvettenträgers (14) höher ist als die Benetzbarkeit der Küvette (19).

10. Verfahren nach Anspruch 9, **dadurch gekennzeichnet, dass** die vorgegebene Flüssigkeitsmenge (23) mittels einer Hohlnadel (27) aus der Zwischenkammer (18) entnommen wird und dass die Hohlnadel (27) gleichzeitig als elektrischer Sensor dient.

## Claims

1. Cuvette module, comprising a cuvette carrier (14) and a cuvette (19) held by the cuvette carrier (14), with an intermediate chamber (18) formed in the cuvette carrier (14), **characterized in that** the cuvette carrier (14) is made of an electrically conductive plastics material, **in that** the cuvette (19) is made of a different material than the cuvette carrier (14), and **in that** the wettability of the cuvette carrier (14) is greater than the wettability of the cuvette (19).

2. Cuvette module according to Claim 1, **characterized in that** the resistivity of the electrically conductive plastics material at room temperature is less than 10⁶ Ω cm.

3. Cuvette module according to Claim 1 or 2, **characterized in that** the cuvette carrier comprises two intermediate chambers lying next to each other, and **in that** the surface resistance of the cuvette carrier (14), measured by two metallic electrodes (30) inserted into the intermediate chambers (18) and in each case in planar contact with three walls of the respective intermediate chamber (18), is between 1 kΩ and 50 kΩ, more preferably between 5 kΩ and 30 kΩ, more preferably between 9 kΩ and 14 kΩ.

4. Cuvette module according to one of Claims 1 to 3, **characterized in that** the electrically conductive plastic is produced from a plastics starting material by using a conductive filler material, wherein the electric conductive plastic has increased wettability by comparison with the plastics starting material.

5. Cuvette module according to Claim 4, **characterized in that** the filler of the electrically conductive plastic is graphite or conductive carbon black.

6. Cuvette module according to one of Claims 1 to 5, **characterized in that** the wetting angle of the cuvette carrier (14) is smaller than the wetting angle of the cuvette (19) by at least 10°, preferably by at least 30°, more preferably by at least 45°.

7. Cuvette module according to one of Claims 1 to 6, **characterized in that** it comprises a plurality of intermediate chambers (18) and a plurality of cuvettes (19).

8. Cuvette module according to one of Claims 1 to 7, **characterized in that** the volume of the intermediate chamber (18) is between 1 µl and 500 µl, preferably between 50 µl and 250 µl.

9. Method for transferring a predefined amount of liquid (23) from a transport container to a cuvette (19), with the following steps:
a. transferring from the transport container to an intermediate chamber (18) an amount of liquid greater than the predefined amount of liquid (23), wherein the intermediate chamber (18) is made of an electrically conductive plastics material and wherein the intermediate chamber (18) is formed in a cuvette carrier (14);
b. determining the filling level in the intermediate chamber (18) by inserting an electrical sensor into the intermediate chamber (18) and establishing a change of the electrical signal when the electrical sensor dips into the liquid;
c. establishing whether the amount of liquid in the intermediate chamber (18) is greater than the predefined amount of liquid (23), and outputting an error message if this is not the case;
d. removing the predefined amount of liquid (23) from the intermediate chamber (18) and transferring the predefined amount of liquid (23) to the cuvette (19), wherein the cuvette (19) is made of a different material than the cuvette carrier (14), and wherein the wettability of the cuvette carrier (14) is greater than the wettability of the cuvette (19).

10. Method according to Claim 9, **characterized in that** the predefined amount of liquid (23) is removed from the intermediate chamber (18) by means of a hollow needle (27), and **in that** the hollow needle (27) serves at the same time as an electrical sensor.

## Revendications

1. Module à cuvette comprenant un porte-cuvette (14) et une cuvette (19) maintenue par le porte-cuvette (14), une chambre intermédiaire (18) étant formée dans le porte-cuvette (14), **caractérisé en ce que** le porte-cuvette (14) se compose d'une matière plastique électriquement conductrice, **en ce que** la cuvette (19) se compose d'un matériau différent de celui du porte-cuvette (14) et **en ce que** la mouillabilité du porte-cuvette (14) est supérieure à la mouillabilité de la cuvette (19).

2. Module à cuvette selon la revendication 1, **caractérisé en ce que** la résistivité de la matière plastique électriquement conductrice à température ambiante est inférieure à 10⁶ Ω.cm.

3. Module à cuvette selon la revendication 1 ou 2, **caractérisé en ce que** le porte-cuvette comprend deux chambres intermédiaires disposées l'une à côté de l'autre et **en ce que** la résistance superficielle du porte-cuvette (14), mesurée avec deux électrodes métalliques (30) qui sont insérées dans les chambres intermédiaires (18) et qui se trouvent respectivement en contact à plat avec trois parois de la chambre intermédiaire (18) correspondante, est comprise entre 1 kΩ et 50 kΩ, de préférence entre 5 kΩ et 30 kΩ, encore de préférence entre 9 kΩ et 14 kΩ.

4. Module à cuvette selon l'une des revendications 1 à 3, **caractérisé en ce que** la matière plastique électriquement conductrice est fabriquée en utilisant une matière de charge conductrice issue d'une matière première plastique, la matière plastique électrique conductrice possédant une mouillabilité accrue en comparaison de la matière première plastique.

5. Module à cuvette selon la revendication 4, **caractérisé en ce que** la matière de charge de la matière plastique électriquement conductrice est du graphite ou du noir de carbone conducteur.

6. Module à cuvette selon l'une des revendications 1 à 5, **caractérisé en ce que** l'angle de mouillage du porte-cuvette (14) et plus petit d'au moins 10°, de préférence d'au moins 30°, encore de préférence d'au moins 45° que l'angle de mouillage de la cuvette (19).

7. Module à cuvette selon l'une des revendications 1 à 6, **caractérisé en ce qu'**il comprend une pluralité de chambres intermédiaires (18) et une pluralité de cuvettes (19).

8. Module à cuvette selon l'une des revendications 1 à 7, **caractérisé en ce que** le volume de la chambre intermédiaire (18) est compris entre 1 µl et 500 µl, de préférence entre 50 µl et 250 µl.

9. Procédé de transfert d'un volume de liquide prédéfini (23) d'un contenant de transport dans une cuvette (19), comprenant les étapes suivantes :
a. transfert d'un volume de liquide, qui est supérieur au volume de liquide prédéfini (23), du contenant de transport dans une chambre intermédiaire (18), la chambre intermédiaire (18) étant composée d'une matière plastique électriquement conductrice et la chambre intermédiaire (18) étant formée dans un porte-cuvette (14) ;
b. détermination du niveau de remplissage dans la chambre intermédiaire (18) en ce qu'un capteur électrique est introduit dans la chambre intermédiaire (18) et une modification du signal électrique est constatée lorsque le capteur électrique est immergé dans le liquide ;
c. constatation si le volume de liquide dans la chambre intermédiaire (18) est supérieur au volume de liquide prédéfini (23) et délivrance d'un message d'erreur si ce n'est pas le cas ;
d. prélèvement du volume de liquide prédéfini (23) hors de la chambre intermédiaire (18) et transfert du volume de liquide prédéfini (23) dans la cuvette (19), la cuvette (19) se composant d'un matériau différent de celui du porte-cuvette (14) et la mouillabilité du porte-cuvette (14) étant supérieure à la mouillabilité de la cuvette (19) .

10. Procédé selon la revendication 9, **caractérisé en ce que** le volume de liquide prédéfini (23) est prélevé de la chambre intermédiaire (18) au moyen d'une aiguille creuse (27) et **en ce que** l'aiguille creuse (27) sert en même temps de capteur électrique.
